# EUROPÄISCHE PATENTSCHRIFT

(11) **EP 1 114 024 B1**
(45) Veröffentlichungstag und Bekanntmachung des Hinweises auf die Patenterteilung: **27.11.2002**
(21) Anmeldenummer: 98942443.7
(22) Anmeldetag: 18.09.1998
(51) Int. Cl.: C07C 311/19, C07D 295/20, C07D 211/60, C07D 211/62, C07D 211/16, C07D 409/12, C07D 295/18, A61K 31/445, A61K 31/50

(54) **UROKINASE-INHIBITOREN**
UROKINASE INHIBITORS
INHIBITEURS DE L'UROKINASE

(43) Veröffentlichungstag der Anmeldung: 11.07.2001
(73) Patentinhaber: Pentapharm A.G., CH-4052 Basel (CH)
(72) Erfinder: WIKSTRÖM, Peter, CH-5073 Gipf-Oberfrick (CH); VIEWEG, Helmut, D-79618 Rheinfelden (DE); STÜRZEBECHER,Jörg, 99094 Erfurt-Rhoda (DE)
(74) Vertreter: Braun, André
(86) Internationale Anmeldenummer: CH9800402
(87) Internationale Veröffentlichungsnummer: WO00017158

(56) Entgegenhaltungen:
- J. STÜRZEBECHER, ET AL.: "Synthesis and Structure-Activity Relationships of Potent Thrombin Inhibitors: Piperazides of Amidinophenylalanine" JOURNAL OF MEDICINAL CHEMISTRY, Bd. 19, Nr. 40, 12. September 1997, Seiten 3091-3099, XP002077904 Washington, DC, US
- F. MARKWARDT, ET AL.: "N-alpha-aryl- sulphonyl-omega-(4-amidinoohenyl)-alpha-am inoalkylcarboxylic acid amides - novel selective inhibitors of thrombin" THROMBOSIS RESEARCH, Bd. 17, Nr. 3-4, 1980, Seiten 425-431, XP002103556 Tarrytown, US in der Anmeldung erwähnt
- J. STÜRZEBECHER, ET AL.: "Inhibition of human mast cell tryptase by benzamidine derivatives" BIOLOGICAL CHEMISTRY HOPPE-SEYLER, Bd. 373, Nr. 10, Oktober 1997, Seiten 1025-1030, XP002103558 Berlin, DE
- G. WAGNER, ET AL.: "Synthese antiproteolytisch wirksamer N-alpha-arylsulfonylierter Amidinophenylglycinamide" DIE PHARMAZIE, Bd. 36, Nr. 7, Juli 1981, Seiten 467-470, XP002103565 Berlin, DE
- J. STÜRZENBECHER, ET AL.: "Synthetische Inhibitoren der Serinproteasen" DIE PHARMAZIE, Bd. 36, Nr. 7, Juli 1981, Seiten 501-502, XP002103566 Berlin, DE

## Beschreibung

Bei der Ausbreitung und Metastasierung solider Tumoren spielen proteolytische Prozesse eine entscheidende Rolle. Zum Auf- und Abbau der Strukturen ihrer unmittelbaren Umgebung verfügen sie neben prokoagulatorischen Substanzen über Enzyme des Fibrinolyse-Systems. Obwohl die (patho)biochemischen Zusammenhänge noch nicht endgültig geklärt sind, kommen dem Plasminogenaktivator Urokinase und dem Urokinase-Rezeptor offenbar eine zentrale Bedeutung zu. Die Entwicklung von Hemmstoffen der Urokinase kann deshalb in erster Linie für die weitere Aufklärung der Rolle von Urokinase und dem Urokinase-Rezeptor bei verschiedenen Krankheiten, speziell bei der Tumorausbreitung und Metastasierung, von grossem Nutzen sein. Daneben stellen Urokinase-Inhibitoren potentielle Arzneimittel zur Beeinflussung der Tumor-Invasion dar.
Urokinase ist ein proteolytisches Enzym und gehört zur Gruppe der Trypsin-ähnlichen Enzyme, die in Proteinen und Peptiden die Bindungen der basischen Aminosäuren Arginin 'und Lysin spalten. Die meisten der heute bekannten Hemmstoffe besitzen deshalb eine stark basische Gruppe, z.B. eine Amidino-Funktion. Erste im mikromolaren Bereich wirksame Hemmstoffe der Urokinase wurden unter Bis-Benzamidinen und unter Verbindungen, die sich vom Naphthamidin ableiten, gefunden (J. Stürzebecher und F. Markwardt, Pharmazie 33, 599-602, 1978). Später wurden Verbindungen mit einer Guanidino-Funktion wie Amiloride (J.-D. Vassalli und D. Belin, FEBS Lett. 214, 187-191, 1987) und Phenylguanidine (H. Yang et al. , J. Med. Chem. 33, 2956-2961, 1990) beschrieben, die Urokinase ebenfalls mit mikromolaren Kᵢ-Werten hemmen. Als sehr wirksame Inhibitoren (Kᵢ bei 0,2µmol/l) wurden Benzothiophen-2-carboxamidine beschrieben (M. J. Towle et al., Cancer Res. 53, 2553-2559, 1993).
Nα-Arylsulfonylierte und Nα-arylsulfonyl-aminoacylierte Derivate des 3-Amidinophenylalanins sind als selektive Hemmstoffe des Thrombins (F. Markwardt et al., Thromb. Res. 17, 425-431, 1980) bzw. von Gerinnungsfaktor Xa (J. Stürzebecher et al., Thromb. Res. 54, 245-252, 1989) bekannt. Wir haben bei der Variation des Nα-Substituenten überraschenderweise gefunden, dass die Einführung eines Triisopropylphenylsulfonyl-Restes die Affinität zu Urokinase ganz entscheidend erhöht. Deshalb stellen Nα-triisopropylphenylsulfonyl- geschützte 3-Amidinophenylalaninderivate eine neue Gruppe von Urokinase-Hemmstoffen dar.

Die vorliegende Erfindung betrifft neue UrokinaseInhibitoren der allgemeinen Formel I, 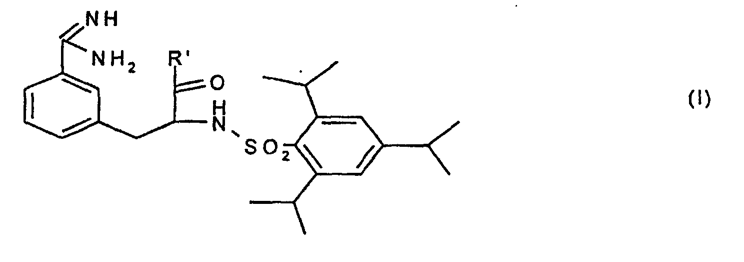 die als Racemate sowie als L- bzw. D-konfigurierte Verbindungen vorliegen und in denen
(a) eine Gruppe der Formel 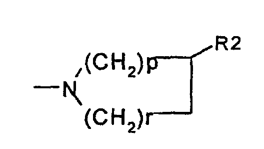 darstellt, in welcher entweder p = 1 und r = 2 und R² Benzyloxycarbonyl, Benzylaminocarbonyl oder 2-Thienylhydrazinocarbonyl sind oder p = 2 und r = 1 und R² Ethoxycarbonyl, 2-Propyloxycarbonyl, 2-Propylaminocarbonyl, Methylaminocarbonyl oder Methyl sind, oder
(b) eine Gruppe der Formel 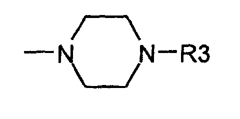 darstellt, in welcher R³ Methoxycarbonyl, Ethoxycarbonyl,Benzyloxycarbonyl, Dimethylaminocarbonyl oder Acetyl ist.

Die Verbindungen liegen in der Regel als Salze mit Mineralsäuren, bevorzugt als Hydrochloride, oder als Salze mit organischen Säuren vor.
Die Verbindungen der allgemeinen Formel I können in prinzipiell bekannter Weise, wie nachfolgend beschrieben, hergestellt werden.
(L)-,(D)- oder (D,L)-3-Cyanphenylalanin-methylesterhydrochlorid wird mit dem entsprechenden Sulfochlorid in Gegenwart einer Base zu einer Verbindung entsprechend der allgemeinen Formel I aber mit Cyanfunktion und in der R¹ = _OCH₃ umgesetzt. Durch milde saure oder alkalische Hydrolyse sind daraus die Verbindungen entsprechend der allgemeinen Formel I aber mit Cyanfunktion und Carbonsäurestruktur (R¹ = -OH) erhältlich. Nach einem in der Peptidchemie üblichen Verfahren, z.B. DCC-Verfahren in Gegenwart von HOBt, sind durch Umsetzung der Carbonsäuren entsprechend der allgemeinen Formel 1 aber mit Cyanfunktion und (R¹ = -OH) mit einem Nucleophil der Struktur (b), die Cyanverbindungen mit entsprechendem R¹ darstellbar. Für die Synthese von Verbindungen mit R¹ = (a) werden zunächst die Carbonsäuren entsprechend der allgemeinen Formel I aber mit Cyanfunktion und R¹ = OH mit Aminosäureestern entsprechend der Struktur (a), wobei aber R² vorzugsweise-COOCH₃ bzw. -COC₂H₅ bedeutet, umgesetzt, die erhaltenen Carbonsäureester unter milden sauren oder alkalischen Bedingungen zu den entsprechenden Carbonsäuren hydrolysiert, die nachfolgend in bereits beschriebener Weise mit entsprechenden Nucleophilen umgesetzt werden können, wobei Verbindungen entsprechend der allgemeinen Formel I mit R¹ = (a) aber mit Cyanfunktion erhalten werden. Die Zielverbindungen der allgemeinen Formel I mit Amidinstruktur sind aus den Cyanverbindungen in bekannter Weise erhältlich, wobei in der Regel durch Addition von H2S an die Cyangruppe zunächst die Thioamide erhalten werden, die durch S-Methylierung. mit Methyliodid in die Thioimidoester und anschliessend durch Behandlung mit Ammoniumacetat in alkoholischer Lösung in die Amidinoverbindungen übergeführt werden. Ausserdem können gegebenenfalls aus den Cyanverbindungen mit Methanol oder Ethanol in Gegenwart von HCl-Gas und in bestimmten Fällen eines inerten Lösungsmittels die entsprechenden Imidoesterhydrochloride dargestellt werden, deren Umsetzung in alkoholischer Ammoniaklösung zu den Amidinoverbindungen führt.
Die erfindungsgemässen Urokinaseinhibitoren können gegebenenfalls zusammen mit mindestens einem geeigneten pharmazeutischen Hilfsstoff zur Herstellung von oral, subkutan oder intravenös verabreichbaren Arzneimitteln zur Tumorbekämpfung oder in der Diagnostik verwendet werden.
Die Arzneimittel zur Tumorbekämpfung bei Menschen und Tieren können oral, subkutan oder intravenös, z.B. in Form von Tabletten, Dragees, Kapseln, Pellets, Suppositorien, Lösungen oder transdermalen Systemen, wie Pflaster, verabreicht werden.

Die Erfindung soll nachfolgend an zwei Beispielen näher erläutert werden.

### Beispiel 1

### N-α-2, 4,6-Triisopropylphenylsulfonyl-(L)-3-amidinophenylalanin-4-ethoxycarbonyl-piperazid-hydrochlorid

### 1.1. N-α-2,4,6-Triisopropyliphenylsulfonyl-(L)-3-cyanphenylalanin-methylester

5 g (L)-3-Cyanphenylalanin-methylester-hydrochlorid wurden in 100 ml Dioxan suspendiert, 4.45 ml NMM zugefügt und 30 min gerührt. Nach Zugabe von 5.97 g 2,4,6-Triisopropylbenzolsulfochlorid in fester Form wurde 3 Tage gerührt, danach ausgefallenes NMM-HCL abfiltriert, das Lösungsmittel abdestilliert und das erhaltene Rohprodukt über KG 60 (Chloroform) gereinigt. Ausbeute: 8.34 g Sirup (90%).

### 1.2. N-α-2,4,6-Triisopropylphenylsulfonyl-(L)-3-cyanphenylalanin

8.34 g der Verbindung 1.1 wurden in einer Mischung aus je 50 ml Essigsäure und 1 N Salzsäure 8 Std. unter Rückfluss erhitzt, nach dem Erkalten 2X mit Ethylacetat extrahiert, die vereinigten Ethylacetatlösungen über M₉SO₄ getrocknet und das Lösungsmittel abdestilliert. Nach Reinigung über KG 60 (Chloroform) wurden 5.8 g eines festen Produktes erhalten (72%).

### 1.3. N-α-2,4,6-Triisopropylphenylsulfonyl-(L)-3-cyanphenylalanin-4-ethoxycarbonyl-piperazid

5.7 g der Verbindung 1.2 wurden in 100 ml THF gelöst, auf O°C abgekühlt, 2.22 g HOBt, 2.82 g DCC zugefügt und 30 min gerührt. Nach Zugabe von 3.94 g 1-Ethoxycarbonyl-piperazin in 30 ml THF wurde über Nacht gerührt, danach ausgefallenes DCU abfiltriert, das Lösungsmittel abdestilliert und das erhaltene Rohprodukt über KG 60 (Chloroform) gereinigt. Ausbeute: 7.1 g eines amorphen Pulvers (96%).

### 1.4. N-α-2,4,6-Triisopropylphenylsulfonyl-(L)-3-amidinophenylalanin-4-ethoxycarbonyl-piperazid-hydrochlorid

7.1 g der Verbindung 1.3 wurden in 30 ml Pyridin gelöst, 30 Tropfen TEA zugefügt, 10 min ein kräftiger Schwefelwasserstoffstrom eingeleitet und 2 Tage bei Raumtemperatur stehengelassen. Anschliessend wurde das Lösungsmittel abdestilliert, der Rückstand in Ethylacetat gelöst, die organische Phase mit 1 N Salzsäure und gesättigter Kochsalzlösung gewaschen, über MgSO₄ getrocknet und das Lösungsmittel abdestilliert. 7.2 g des auf diese Weise erhaltenen Thioamids wurden in 250 ml Aceton gelöst, die Lösung mit 17 g Methyliodid versetzt und 2 Tage bei Raumtemperatur unter Lichtschutz stehengelassen. Danach wurde das Lösungsmittel abdestilliert, das Thioimidesterhydroiodid (8.5 g) in 50 ml Methanol gelöst, 1.9 g Ammoniumacetat zugefügt und der Ansatz 4 Std. bei 60°C erwärmt. Das nach Abdestillieren des Lösungsmittels erhaltene Rohprodukt wurde über LH20 Methanol) gereinigt. Das auf diese weise erhaltene Amidin-hydroiodid wurde über Ionenaustauscher (Amberlite IRA-420) in das Hydrochlorid übergeführt. Ausbeute: 5.3 g eines amorphen Pulvers (69%).

### Beispiel 2

### Nα-2,4,6-Triisopropylphenylsulfonyl-(D,L)-3-amidinophenylalanyl-nipecotinsäure-benzylamid-hydrochlorid

### 2.1. Nα-2,4,6-Triisopropylphenylsulfonyl-(D,L)-3-cyanphenylalanyl-nipecotinsäure-ethylester

4.57 g Nα-2,4,6-Triisopropylphenylsulfonyl-(D,L)-3-cyanphenylalanin (aus (D,L)-3-Cyanphenylalaninmethylesterhydrochlorid und dem entsprechenden Sulfochlorid analog 1.1 und 1.2 dargestellt), 1.5 g HOBt und 2.42 g DCC wurden in 50 ml DMF gelöst, 1 Std. gerührt und danach 2.36 g Nipecotinsäure-ethylester zugefügt. Nach Rühren über Nacht wurde ausgefallenes DCU abfiltriert, das Lösungsmittel abdestilliert, der Rückstand in wenig Methanol gelöst und zur Kristallisation stehengelassen. Der gebildete Niederschlag wurde abgesaugt, mit Methanol gewaschen und getrocknet. Ausbeute: 4.46 g (75%).

### 2.2. Nα-2,4,6-Triisopropylphenylsulfonyl-(D,L)-3-cyanphenylalanyl-nipecotinsäure

4.4 g des vorher beschriebenen Ethylesters wurden in einer Mischung aus 35 ml Essigsäure und 25 ml 1 N HCl 2 Std. unter Rückfluss erhitzt. Nach Zugabe von 10 ml Wasser wurde zum Erkalten stehengelassen, wobei sich ein wachsartiges Produkt abschied. Nach Abgiessen des Lösungsmittels wurden 200 ml Wasser zugesetzt, längere Zeit kräftig gerührt und die erhaltene feste Substanz abgesaugt, mit Wasser gewaschen und getrocknet. Ausbeute: 3.84 g (92%).

### 2.3. Nα-2,4,6-Triisopropylphenylsulfonyl-(D,-L)-3-cyanphenylalanyl-nipecotinsäure-benzylamid

2.28 g der vorher beschriebenen Verbindung, 0.6 g HOBt und 0.97 g DCC wurden in 20 ml DMF gelöst, 1 Std. gerührt, anschliessend 0.6 g Benzylamin zugefügt und weiter über Nacht gerührt. Nach Abfiltrieren des ausgefallenen DCU wurde das Lösungsmittel abdestilliert, der Rückstand in Methanol gelöst und die Lösung in 5-proz. Natriumhydrogencarbonatlösung/Eis gegossen. Nach 1 Std. wurde der gebildete Niederschlag abgesaugt, mit Wasser gewaschen und im Vakuum getrocknet. Ausbeute: 2.48 g (94%).

### 2.4. Nα-2,4,6-Triisopropylphenylsulfonyl-(D,L)-3-amidinophenylalanyl-nipecotinsäure-benzylamid-hydrochlorid

2.4 g der Verbindung 2.3 wurden in 30 ml Pyridin gelöst, 30 Tropfen TEA zugefügt, in die Lösung 10 Min. Schwefelwasserstoff eingeleitet und der Ansatz 2 Tage bei Raumtemperatur stehengelassen. Anschliessend wurde das Lösungsmittel abdestilliert, der Rückstand in Ethylacetat aufgenommen und mit 1 *N* HCl ausgeschüttelt. Nach Waschen der organischen Phase mit gesättigter Kochsalzlösung und Trocknen über Natriumsulfat wurde das Lösungsmittel abdestilliert. 2.38 g des auf diese Weise erhaltenen Thioamids wurden in 100 ml Aceton gelöst, die Lösung mit 6.5 g Methyliodid versetzt und 20 Std. bei Raumtemperatur unter Lichtschutz stehengelassen. Danach wurde das Lösungsmittel abdestilliert, das Thioimidoester-hydroiodid in 50 ml Methanol gelöst, 0.5 g Ammoniumacetat zugegeben und der Ansatz 4 Std. bei 60°C im Wasserbad erwärmt. Das nach Abdestillieren des Lösungsmittels erhaltene Rohprodukt konnte über KG 60 gereinigt werden. Die Elution erfolgte zunächst mit Chloroform, danach mit Chloroform/Methanol 9:1. Das so gereinigte Amidin-hydroiodid wurde über Ionenaustauscher (Amberlite IRA-420) in das Hydrochlorid übergeführt. Ausbeute: 1-45 g eines amorphen Pulvers (56%).

Die Charakterisierung der Verbindungen erfolgte massenspektrometrisch, eine Reinheitsprüfung erfolgte mittels DC und HPLC.

### Abkürzungen

- NMM: N-Methylmorpholin
- KG 60: Kieselgel 60
- THF: Tetrahydrofuran
- HOBt: 1-Hydroxy-benzotriazol
- DCC: Dicyclohexylcarbodiimid
- DCU: Dicyclohexylurea
- LH 20: Sephadex LH 20
- DC: Dünnschichtchromatographie
- HPLC: Hochdruckflüssigkeitschromatographie

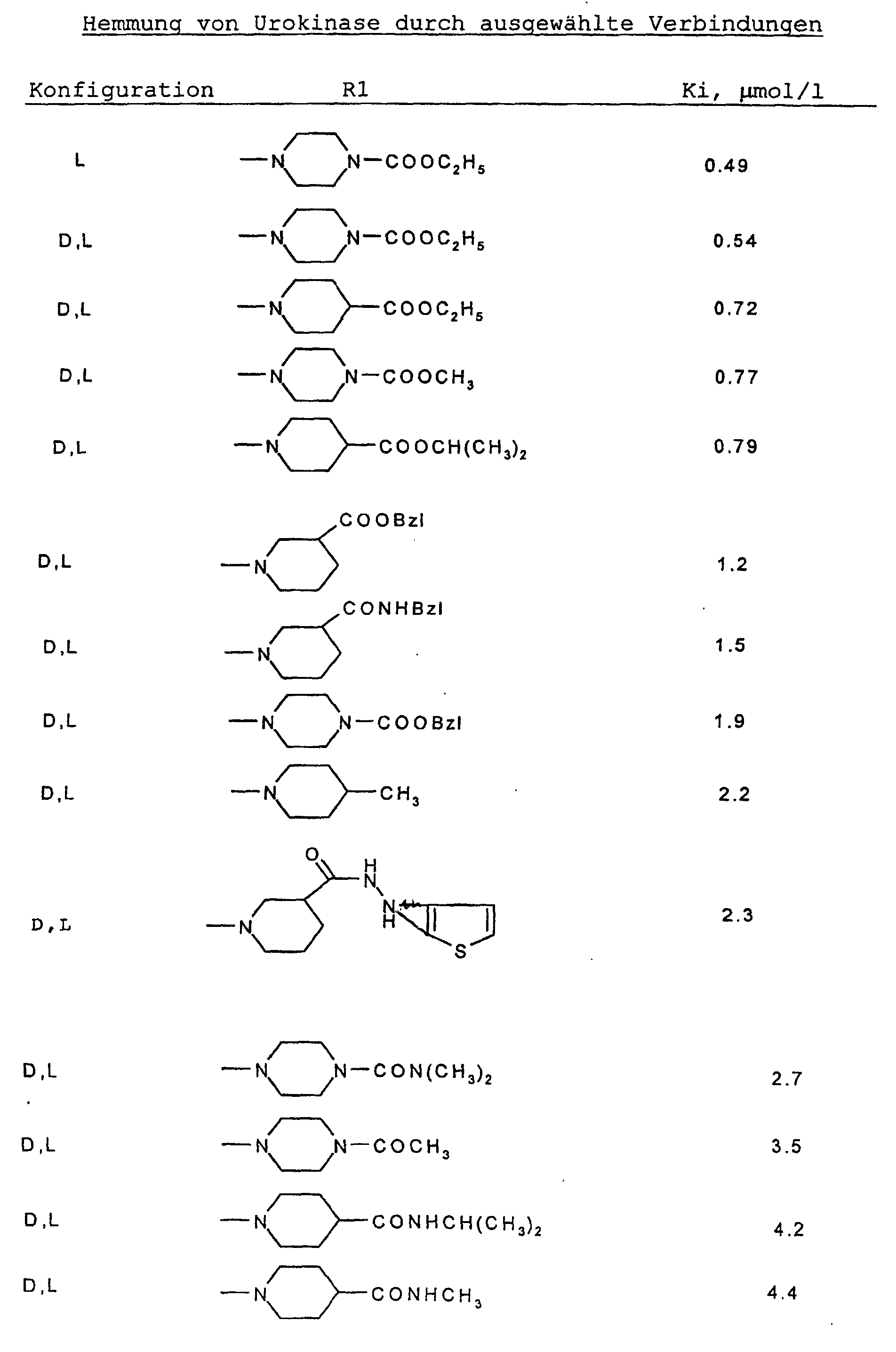

### Abkürzung: Bzl = Benzyl

### Bestimmung der Hemmwirkung

Zur Bestimmung der Inhibitoraktivität wurden 200 _1 Tris-Puffer (0,05 mol/l, den Inhibitor enthaltend, 0,154 mol/l NaCl, 5% Ethanol, pH 8,0), 25 _1 Substrat (Pefachrome UK oder Bz-ßAla-Gly-Arg-pNA in H₂O; Pentapharm Ltd., Basel, Schweiz) und 50 _1 sc-Urokinase (Ribosepharm GmbH, Haan, Deutschland) bei 25°C inkubiert. Nach 3 min wurde die Reaktion durch Zugabe von 25 _1 Essigsäure (50%) unterbrochen und die Absorption bei 405 nm mittels Microplate Reader (MR 50001 Dynatech, Denkendorf, Deutschland) bestimmt. Die Kᵢ-Werte wurden nach Dixon durch lineare Regression mittels eines Computerprogramms ermittelt. Die Kᵢ-Werte sind das Mittel aus mindestens 3 Bestimmungen, die Standardabweichung lag unter 25%.

## Patentansprüche

1. Neue Urokinase-Inhibitoren der allgemeinen Formel I, 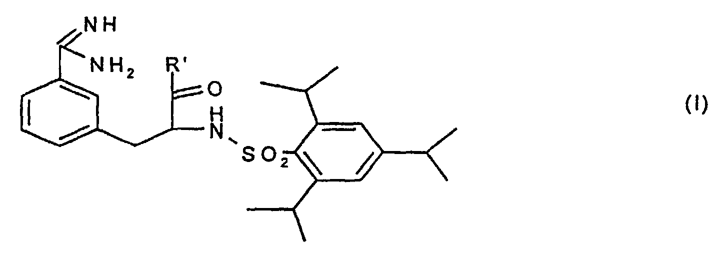 die als Racemate sowie als L- bzw. D-konfigurierte Verbindungen vorliegen und in denen R'
(a) eine Gruppe der Formel 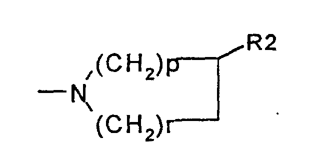 darstellt, in welcher entweder p = 1 und r = 2 und R² Benzyloxycarbonyl, Benzylaminocarbonyl oder 2-Thienylhydrazinocarbonyl sind oder p = 2 und r = 1 und R² Ethoxycarbonyl, 2-Propylycarbonyl, 2-Propylaminocarbonyl, Methylaminocarbonyl oder Methyl sind; oder
(b) eine Gruppe der Formel 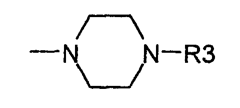 darstellt, in welcher R³ Methoxycarbonyl, Ethoxycarbonyl, Benzyloxycarbonyl, Dimthylaminocarbonyl oder Adtyl ist,
wobei die Verbindungen in Form ihrer freien Basen als auch als Salze mit Mineralsäuren, bevorzugt als Hydrochloride, oder als Salze mit organischen Säuren vorliegen.

2. Verwendung der Urokinaseinhibitoren nach Patentanspruch 1 zur Herstellung von oral, subkutan oder intravenös verabreichbaren Arzneimitteln zur Tumorbekämpfung.

3. Oral, subkutan oder intravenös verabreichbares Arzneimittel zur Tumorbekämpfung, enthaltend mindestens einen Inhibitor nach Patentanspruch 1 und gegebenenfalls einen oder mehrere pharmazeutische Hilfsstoffe.

4. Arzneimittel nach Patentanspruch 3 in Form von Tabletten, Dragees, Kapseln, Pellets, Suppositorien, Lösungen oder transdermalen Systemen, wie Pflaster.

5. Verwendung der Urokinaseinhibitoren nach Patentanspruch 1 zur Herstellung eines Mittels für die Diagnostik.

6. Verwendung der Urokinasehemmer gemäss Patentanspruch 1 zur Herstellung eines Arzneimittels zur Urokinasehemmung bei Lebewesen, insbesondere beim Menschen.

## Claims

1. New urokinase inhibitors of general formula I 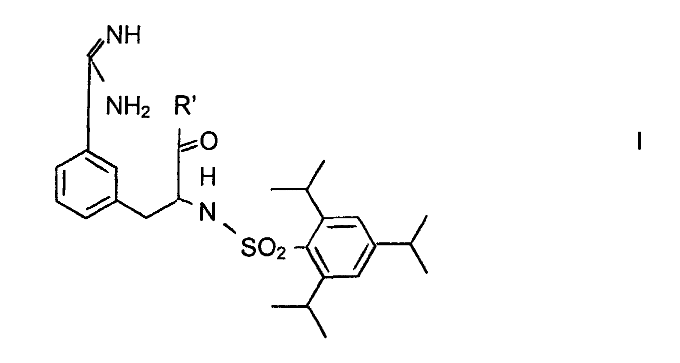 which are present as racemates as well as compounds in L- or D-form, respectively, and wherein R' represents
(a) a group of formula 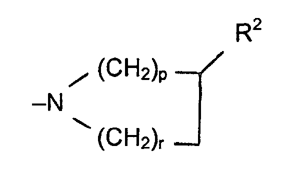 wherein either p = 1 and r = 2 and R² represents benzyloxycarbonyl, benzylaminocarbonyl or 2-thienylhydrazinocarbonyl, or p = 2 and r = 1 and R² represents ethoxycarbonyl, 2-propyloxycarbonyl, 2-propylaminocarbonyl, methylaminocarbonyl or methyl; or
(b) a group of formula 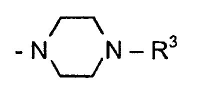 wherein R³ represents methoxycarbonyl, ethoxycarbonyl, benzyloxycarbonyl, dimethylaminocarbonyl or acetyl,
the compounds being present in the form of their free bases or as salts with inorganic acids, preferably hydrochlorides, or as salts with organic acids.

2. Use of the urokinase inhibitors according to claim 1 for the manufacture of orally, subcutaneously or intravenously administrable drugs for combating tumors.

3. Orally, subcutaneously or intravenously administrable drug for combating tumors, containing at least one inhibitor according to claim 1 and possibly one or several pharmaceutical additives.

4. Drug according to claim 3 in the form of tablets, dragées, capsules, pellets, suppositories, solutions or transdermal systems, such as plasters.

5. Use of the urokinase inhibitors according to claim 1 for the manufacture of a preparation to be used in diagnosis.

6. Use of the urokinase inhibitors according to claim 1 for the manufacture of a drug for urokinase inhibition in living organisms, in particular in humans.

## Revendications

1. Nouveaux inhibiteurs d'urokinase de formule générale I 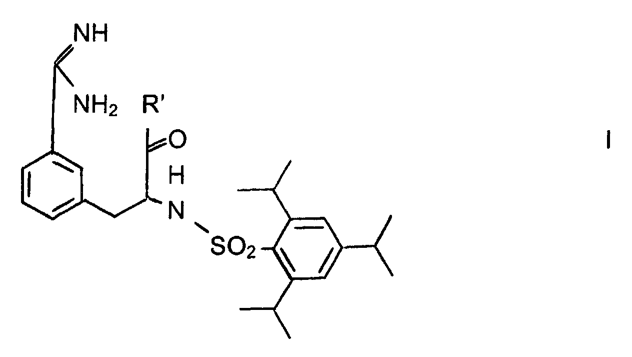 se présentant sous forme de composés racémiques ou de configuration L ou D et dans lesquels R' représente
(a) un groupement de formule 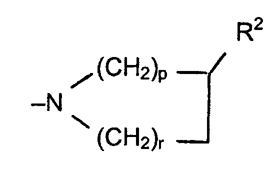 où soit p = 1 et r = 2 et R² représente du benzyloxycarbonyle, benzylaminocarbonyle ou 2-thienylhydrazinocarbonyle, soit p = 2 et r = 1 et R² représente de l'éthoxycarbonyle, 2-propyloxycarbonyle, 2-propylaminocarbonyle, méthylaminocarbonyle ou méthyle; ou
(b) un groupement de formule 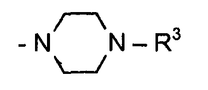 où R³ représente du méthoxycarbonyle, éthoxycarbonyle, benzyloxycarbonyle, diméthylaminocarbonyle ou acétyle,
les composés se présentant sous la forme de leurs bases libres ou sous forme de sels avec des acides minéraux, de préférence des hydrochlorures, ou de sels avec des acides organiques.

2. Utilisation des inhibiteurs d'urokinase selon la revendication 1 pour la fabrication de médicaments contre les tumeurs pouvant être administrés par voie orale, sous-cutanée ou intraveineuse.

3. Médicament contre les tumeurs pouvant être administré par voie orale, sous-cutanée ou intraveineuse et contenant au moins un inhibiteur selon la revendication 1 et éventuellement un ou plusieurs excipients pharmaceutiques.

4. Médicament selon la revendication 3 se présentant sous forme de comprimés, dragées, capsules, pilules, suppositoires, solutions ou systèmes transdermiques, tels que des emplâtres.

5. Utilisation des inhibiteurs d'urokinase selon la revendication 1 pour la fabrication d'un produit diagnostique.

6. Utilisation des inhibiteurs d'urokinase selon la revendication 1 pour la fabrication d'un médicament destiné à inhiber l'urokinase dans les organismes vivants, en particulier chez l'homme.
